# EUROPEAN PATENT SPECIFICATION

(11) **EP 2 753 181 B1**
(45) Date of publication and mention of the grant of the patent: **26.09.2018**
(21) Application number: 12783651.8
(22) Date of filing: 30.08.2012
(51) Int. Cl.: C12R 1/07, A01N 63/00, A01P 3/00

(54) **USE OF A COPPER RESISTANT, FENGYCIN-PRODUCING BACILLUS MOJAVENSIS STRAIN FOR CONTROLLING VEGETABLE PATHOGENS**
VERWENDUNG EINES KUPFER-RESISTENTEN, FENGYCIN-PRODUZIERENDER BACILLUS MOJAVENIS STAMM ZUR KONTROLLE PFLANZLICHER PATHOGENE
UTILISATION D'UNE SOUCHE DE BACILLUS MOJAVENSIS PRODUISANT DE LA FENGYCINE RÉSISTANTE AU CUIVRE POUR RÉGULER LES PATHOGÈNES DES LÉGUMES

(30) Priority: 08.09.2011 HU P1100498
(43) Date of publication of application: 16.07.2014
(73) Proprietor: Szegedi Tudományegyetem, 6720 Szeged (HU)
(72) Inventor: MANCZINGER, László, H-6791 Szeged (HU); VÁGVÖLGYI, Csaba, H-6771 Szöreg (HU); SAJBEN, Enikö, H-6726 Szeged (HU); NAGY, Árpád, H-6726 Szeged (HU); SZÖKE-KIS, Zoltán, H-5650 Mezöberény (HU); NAGY, Adrienn, H-6000 Kecskemét (HU); TURÓCZI, György, H-2100 Gödöllö (HU); KOVÁCS, András, H-6000 Kecskemét (HU)
(74) Representative: Molnar, Istvan
(86) International application number: PCT/HU2012/000084
(87) International publication number: WO 2013/034938

(56) References cited:
- WO-A1-2011/121408
- US-A1- 2007 224 179
- US-A1- 2010 092 442
- VAGVOLGYI CSABA ET AL: "THE EFFECT OF COPPER ON THE EFFECTIVENESS OF BIOCONTROL BACTERIUM STRAINS", CEREAL RESEARCH COMMUNICATIONS, SZEGEL, HU, vol. 37, no. Suppl. S, 1 January 2009 (2009-01-01), pages 589-592, XP009167229, ISSN: 0133-3720

## Description

### Brief description of the invention

The invention relates to a biological material for exerting antagonism against vegetable pathogens, which contains *Bacillus mojavensis* R3B mutant strain deposited under # NCAIM (P) B 001389 according to the Budapest Treaty. The biological material according to the invention is an effective antagonist against the pathogens of vegetables, preferably tomato, pepper, lettuce and/or cabbage, in particular against the pathogens selected from the group of *Xanthomonas vesicatoria, Pseudomonas syringae* and *Clavibacter michiganensis* vegetable pathogen bacteria and *Pythium debaryanum, Phytophthora infestans, Alternaria alternata* and *Fusarium oxysporum* vegetable pathogen fungi. Furthermore, the invention relates to a composition comprising the biological material according to the invention and optionally a copper-containing pesticide and a process for controlling vegetable pathogens, furthermore use of the biological material or composition according to the invention for the protection of vegetables, preferably tomato, pepper, lettuce and/or cabbage.

### Background of the invention

The effective pest control is part of the intensive vegetable production technologies, however, the expectations of the consumer markets, furthermore the efforts to improve the food safety called for the minimization of pesticides, and replacing them by biological methods. In a closed area production the entire biological control has been achieved against insect pests, however, the biological control of bacteria and fungi is still to be elaborated.

Nowadays there is an increasing need in the fields of vegetable production and sales for bioproducts containing no residues of chemical pesticides. Among the effective chemical pesticides only the inorganic copper-compositions are allowed in the course of production of bioproducts. The number of copper resistant mutants among the vegetable pathogen microorganisms has increased a lot in the last couple of years, thus these copper containing agents alone are insufficient to achieve effective and reliable pest control. One possible solution to overcome this problem is to effect an integrated pest control using copper containing agents and biocontrol compositions. Biocontrol products may also be used alone, but in such cases the highest efficiency of the best compositions is still under 50 percent.

The majority of the antibacterial or antifungal biocontrol products presently available in the world market contain one component as active agent, which is a bacterium or fungi possessing antagonist and/or parasite features. The *Bacillus* strain has long been used for vegetable pest control purposes. The compositions sold are especially effective against vegetable pathogen fungi. The compositions already been commercialized and patented comprise mainly the strains of *Bacillus subtilis* and *Bacillus amiloliquefaciens* species. Their effectiveness is explained by different peptide antibiotics, secreted outside of the cell walls, which are effective mainly against fungi: surfactin, iturin, fengycin, bacillomycin, mycosubtilin. Recently it has been suggested that their effectiveness is enhanced by extracellular enzymes decomposing the cell walls and/or the membrane of the cytoplasm of the pathogen microorganism: proteases, kitinases and lipases. Besides, several independent investigations have proved that of the secreted antibiotics mainly fengycin is responsible for exerting of the induced resistance response in the treated plants

### The state of the art

US patent No. 2010092442 relates to a process to induce an acquired systemic resistance of plants against infections. The method is based on the utilization of a composition comprising bacteria belonging to the *Bacillus* strain, as a consequence of which the plant produces protecting proteins. The applied bacteria are the isolate of *Bacillus mojavensis* 203-7-, and the isolate of *Bacillus mycoides* BmJ. The drawback of the *Bacillus mojavensis* isolate are on one hand that the application of the given *Bacillus mojavensis* 203-7 isolate and the antibacterial agent together is not proposed, on the other hand, the isolated strain is not copper resistant, thus the combined application thereof with copper-containing pesticides would not achieve the expected pest control effect. Furthermore, the *Bacillus mojavensis* 203-7 isolate disclosed in the patent has no fengycin hyper-producing properties.

International patent application No. WO9724433 discloses biocontrol methods and microorganisms, which are capable of controlling plant diseases of fungi origin. The microorganisms useful for biocontrol purposes are novel endophyte symbiotic *Enterobacter cloacae* strains. Said strains are suitable for passing useful genetic products into the plants. The used Enterobacter cloacae strains are rifampicin resistant. The *Enterobacter cloacae* strains according to the patent application (deposited under No. ATCC55732) as analysed their 16S rRNS gene sequence have proven to be *Bacillus mojavensis* strains. The shortcoming of the isolate according to the patent application is that it has no copper resistance, therefore its co-application with copper containing pesticides is likely to be discouraged.

International patent application No. WO9909834 discloses compositions and methods for the treatment of arable soil, which are capable of improving the growth of plants and the results of reaping. The composition is based on pesticide resistant microorganism strains. One strain or a mixture of several strains among the microorganisms in the composition is applied in the arable soil attached to the seeds of plants. The microorganisms of the composition are selected from *Azospirillum lipoferum ssp.* lip7R 885, *Azospirillum amazonense ssp.* K21R 887, *Azospirillum irakense ssp.* 5041R 889, *Azospirillum brasilense ssp.* A41R 879, *Azotobacter vinelandii ssp.* ESZ 2132, *Pseudomonas sp.* Szeged 344 O.P. 14, *Pseudomonas fluorescens* var. MOB24, Res24, *Bacillus circulans* var. Res. 97, *Bacillus megaterium* var. Res. 54, *Rhizobium meliloti* var. PolRes. 7, *Alcaligenes faecalis* var. Res36 and PhylO6-R+32. The strains of the bacteria had been isolated from the environment of different plants and dissimilar soils, then have been made pesticide resistant. The object of the solution according to the patent application is other then the antagonism against the pests of green vegetables.

International patent application No. WO9821964 relates to an individual *Bacillus subtilis* strain, which is capable of inhibiting plant pathogen fungi and bacteria. The *Bacillus subtilis* strain according to the prior art document capable of protecting different plants (fruits, green vegetables) from the infections caused by the pathogens selected from the bacteria and *Botrytis, Fusarium, Phytophthora, Pseudomonas, Erwinia, Alternaria, Trichoderma, Monilinia, Puccinia, Rhizoctonia, Phythium* and *Plasmopara.* The strain may be applied together with pesticides as well, however suffers from the drawback of lacking copper resistance, therefore the application of copper containing pesticides is excluded in this case.
Vágvölgyi et al. [Cereal Research Communications, Szeged, HU, vol. 37 no. Suppl. S, (2009-01-01), pages 589-592] discloses three *Bacillus mojavensis* strains, which show high copper tolerance being tested on e.g. *Alternaria solani* and *Pythium ultimum.* However, these *Bacillus mojavensis* strains are different biological entities from that provided by our invention, namely *Bacillus mojavensis* R3B mutant strain, which mutant strain shows antagonist effect against e.g. *Alternaria alternata* and *Pythium debaryanum,* and which mutant strain spontaneously appearing after transferring of the *Bacillus mojavensis* B5 strain onto a culturing medium containing 400 µg/ml copper sulphate, as disclosed as disclosed below.
It can be seen from the above that the solutions according to the state of the art disclose bacteria relevant from the pest control point of view, which can be applied together with pesticides, however, they fail to disclose a copper resistant bacterium strain, which would enable one to use further copper containing pesticides in order to achieve more effective plant protection. Furthermore, there is need for compositions, with which an effective control of the copper resistant plant pathogen bacteria would be achieved. There is need therefore for novel compositions against plant pathogen bacteria and fungi, which would overcome the drawback of the state of the art, especially the application of complicated, multiple step and environmentally stressing pest control technologies. In order to meet these needs we have done systematic research and development, as a result of which we have completed our invention.

### Detailed description of the invention

### Definitions

The term "pest" or "pathogen" as used in the present description means a virus, bacterium or fungus, which lives parasitically in different living organisms, and settling and reproducing in the host or its body (e.g. in a human) causes a disease. The measure of the ability to infect, the pathogenity, may be variable even within one species.
The term "antagonist effect" or "antagonism" as used in the present description means a relationship between microorganisms, in which the members of one species are killed, deterred, or their reproduction is inhibited by the representatives of another species, e.g. through the chemicals (e.g. antibiotics, extracellular enzymes) produced by them.
The term "pest controlling agent" or "pesticide" as used in the present description means a plant protecting agent (chemicals, other agents and a combination of them), whose application intends to kill, exclude, warn off, inhibit or any kind of control pathogen organisms, thereby the effective protection of plants. The pesticide according to the invention may be selected by the skilled person on considering health and safety issues, without undue experimentation.
The term "dose formulation" as used in the present description characterises the type of formulation according to the composition, which is determined according to the Pesticide formulation types and international coding system catalogue (Crop-Life International Technical Monography, No. 2; 5th Edition, 2002). This may be e.g. aqueous suspension, suspension concentrate, capsulated concentrate, emulsion forming liquid spray, granule, granule dispersible in water, microgranule, water soluble powder, but is not limited thereto. The dose formulation which may be used according to the invention may be selected by the skilled person without undue experimentation.

The term "copper containing pesticide" as used in the present description means a pest control composition, which contains copper. The meaning of the copper containing pesticide is not particularly limited, and it may be selected from the group of copper-sulphate, copper-oxyquinolate, copper-oxide, copper-hydroxide and copper-oxy-chloride. The copper containing pesticide according to the invention may be selected by the skilled person without undue experimentation.

The term "excipient" as used in the present description is not particularly limited, and the excipients may be selected from the group as follows:
a) in case of a liquid formulation e.g. water or an organic solvent (e.g. xilene, methanol, ethylene-glycol or mineral oil), a dispersion stabilizator, a surfactant (e.g. calcium-dodecyl-benzene-sulphonate, polyglycol-ether, etoxylated alkyl-phenol or alkyl-aryl-sulphonates), optionally waxes,
b) in case of a granular formulation montmorillonite, bentonite, wood flour, starch, cellulose and a binder, such as e.g. a mineral oil, polyvinyl-alcohol or saccharose,
c) and other in itself known, usual additive and/or excipient.

The excipient according to the invention may be selected by the skilled person without undue experimentation.

### The discovery according to the invention

As a result of the systematic experimental work directed to the present invention we have surprisingly found that the *Bacillus mojavensis* R3B mutant strain
a) exerts stronger antagonist effect against pathogens,
b) carries copper resistance and
c) induces resistance against pathogens in plants
   unlike/as compared to the state of the art.

### Detailed description of the invention

Based on the above, the present invention relates in its first aspect to a biological material for exerting antagonism against vegetable pathogens, which contains *Bacillus mojavensis* R3B mutant strain deposited under # NCAIM (P) B 001389 according to the Budapest Treaty. The biological material according to the invention is an effective antagonist against the pathogens of vegetables, preferably tomato, pepper, lettuce and/or cabbage, in particular against the pathogens selected from the group of *Xanthomonas vesicatoria, Pseudomonas syringae* and *Clavibacter michiganensis* vegetable pathogen bacteria and *Pythium debaryanum, Phytophthora infestans, Alternaria alternata* and *Fusarium oxysporum* vegetable pathogen fungi.

Although we do not wish to restrict the explanation of the antagonist effect according to the present invention to one theory, it can be seen that the unique effect of the biological material according to the invention against the pathogens is on one hand inhibits the operation of pathogens in the rhysosphere of the plants and on the other hand, at the same time it induces resistance in the protected plant against the inhibited pathogens.

In the second aspect, the present invention relates to a composition that comprises a culture of the biological material according to the invention and optionally a copper-containing pesticide, preferably copper-sulphate, copper-oxyquinolate, copper-oxide, copper-hydroxide or copper-oxy-chloride, and optionally an excipient.

Based on the above, the copper-containing pesticide according to the present invention is not particularly limited, in condition that it does not kill the biological material according to the invention, or does not inhibit its operation to an unwanted extent, and such useful copper-containing pesticides include copper-sulphate, copper-oxyquinolate, copper-oxide, copper-hydroxide or copper-oxy-chloride, but are not limited thereto. The pesticide according to the invention may be selected by the skilled person without undue experimentation.

Based on the above, the excipient according to the present invention is not particularly limited, in condition that it does not decrease the effectiveness of the biological material according to the present invention as an active agent, or a biological and chemical active agent combination, and the applicable excipients include without limitation the following:
a) in case of a liquid formulation e.g. water or an organic solvent (e.g. xilene, methanol, ethylene-glycol or mineral oil), a dispersion stabilizator, a surfactant (e.g. calcium-dodecyl-benzene-sulphonate, polyglycol-ether, etoxylated alkyl-phenol or alkyl-aryl-sulphonates), optionally waxes,
b) in case of a granular formulation montmorillonite, bentonite, wood flour, starch, cellulose and a binder, such as e.g. a mineral oil, polyvinyl-alcohol or saccharose,
c) and other in itself known, usual additive and/or excipient.

The excipient according to the invention may be selected by the skilled person without undue experimentation.

The dose form according to the present invention is not particularly limited, provided that it is suitable for the application of the biological material according to the invention as active agent, or the composition containing said biological material according to the invention to the protected plant or any part thereof. Such applicable dose forms include without limitation the following: aqueous suspension, suspension concentrate, capsulated concentrate, emulsion forming liquid spray, granule, granule dispersible in water, microgranule, water soluble powder. The dose formulation which may be used according to the invention may be selected by the skilled person without undue experimentation.

In its third aspect the invention relates to a process for controlling vegetable pathogens according to which the biological material or composition according to the invention is applied to a plant, preferably to a vegetable, more preferably to tomato, pepper, lettuce and/or cabbage. The biological material according to the invention is preferably applied to the seeds of the protected plant, roots of the protected plant, stem of the protected plant, leaves of the protected plant, blooms of the protected plant, the foliage of the protected plant or fruits of the protected plant, is mixed to the irrigation water of the plant and/or sprayed to the protected plant.

Finally, the invention relates to the use of the biological material or composition according to the invention for the control of pests, preferably for the control of the pests of vegetables, more preferably for the control of the pests of tomato, pepper, lettuce and/or cabbage, furthermore, for inducing resistance in said plants against the pathogens according to the present invention.

### Examples

In the following, our invention is further detailed through preparation and working examples, referring to the figures listed below, annexed to the description.
Figure 1 shows the antagonist activity of 20 bacterium strains as a function of the copper concentration.
Figure 2 shows the change of the antagonist effect against fungi as a function of the copper concentration, an average of 20 strains.
Figure 3 shows the thin layer chromatograpy of the antibiotics spectrum secreted by the *Bacillus mojavensis* B5 strain.
Figure 4 shows the antibiotics production kinetics of the *Bacillus mojavensis* B5 strain in a fermentor.
Figure 5 shows the ability of the *Bacillus mojavensis* B5 and the copper-resistant mutants made therefrom to produce extracellular protease.
Figure 6 shows the inhibition of *Pseudomonas syringae* by the a *Bacillus mojavensis* B5 and the copper-resistant mutants made therefrom.

### The isolation of Bacillus mojavensis B5 strain

In the course of our experiments we have tested the antagonist ability of 82 endophyte bacterium strains and 44 bacterium strains isolated from the rhysosphere, then we have selected the best 20 antagonist strains, and their antagonist abilities have been tested in the presence of copper. Among the best antagonist 10 copper-resistant Bacillus strains, the *Bacillus mojavensis* B5 strain possessed the best antagonist features, especially the R3B copper-resistant mutant strain, which is the subject matter of our claim for the protection.

### The isolation of dominant bacterium strains from the rhysosphere and roots of the produced plants, and testing of their antagonism

The isolations were made from the root surface and rhizomes of different tomato and pepper species on bacterium selective culturing medium. 10- 10 strains were isolated from the dominant colony types in case of each tested sample. The best antagonists were selected against *Pseudomonas syringae, Xanthomonas vesicatoria, Erwinia carotovora,* and *Phytophthora infestans, Sclerotinia sclerotiorum, Alternaria solani* and *Botrytis cinerea* with preliminary antagonism tests on culturing plates. These were identified on species level by partial sequencing their 16S RNS gene, in order to exclude the plant pathogens from the further examinations. The efficacy of the non pathogenic strains was tested against another plant pathogenic bacteria and fungi. 40 of the best antagonists were selected for the *in vivo* plant treatment examinations.

### Isolation and testing of the endophyte bacteria

82 endophyte bacterium strains were isolated from the roots and seeds of different vegetables (parsley, carrot, tomato, pepper, white cabbage, lettuce, spring onion, cucumber). Their ability to antagonize the plant pathogen *Fusarium oxysporum, Rhizoctonia solani; Xanthomonas campestris pv vesicatoria, Erwinia carotovora* and *Pseudomonas syringae* strains was tested by *in vitro* antagonism tests. The antagonist potential of the best 10 strains was tested against the plant pathogen fungi *Phytophtora infestans, Botrytis cinerea, Sclerotinia sclerotiorum* and *Alternaria tenuis* as well. Furthermore, the effect of pH on the growth was tested: it was determined, which strains can grow at pH=5.5. This has importance in their applicability in more acidic soils. Preliminary copper tolerance test were made with the strains (this is important for the reason of their applicability in the integrated pest control) with culturing media containing 25 and 50 microgram/ml CuSO₄, and found that 71 and 49 strains grew in the tested values. To determine the strains, the 16S RNS gene was amplified by PCR (primers: Eub8F and Eub534R), sequenced, then compared with the databases to exclude the plant or human pathogen strains. 10 good antagonist bacteria were used for the plant tests out of the 82 original endophyte bacteria.

### The examination of the bacterium strains isolated from the rhyzosphere

Samples were taken from a greenhouse, from the rock wool of tomato plants grown in a hydroponic system, and 39 strains were isolated on a culture medium buffered to pH=5.5. Their antagonism was tested against *Fusarium oxysporum, Phytophtora infestans, Botrytis cinerea, Sclerotinia sclerotiorum, Alternaria tenuis, Clavibacter michiganense, Xanthomonas campestris pv vesicatoria* and *Pseudomonas syringae.*

From 10 samples originating from arable soil 44 strains were isolated on a pH=5.5 culture medium, 28 strains with the ability to grow in cold conditions, and 27 strains on a *Pseudomonas* selective culturing medium using Bacillus strain selective methods. These strains were tested against *Clavibacter michiganense, Xanthomonas campestris pv vesicatoria* and *Pseudomonas syringae* by *in vitro* antagonism tests. To determine the strains, the 16S RNS gene was amplified by PCR (primers: Eub8F and Eub534R), sequenced, then compared with the databases to exclude the plant or human pathogen strains.

### The preparation of copper-resistant mutants from the bacterium strains showing excellent antagonism

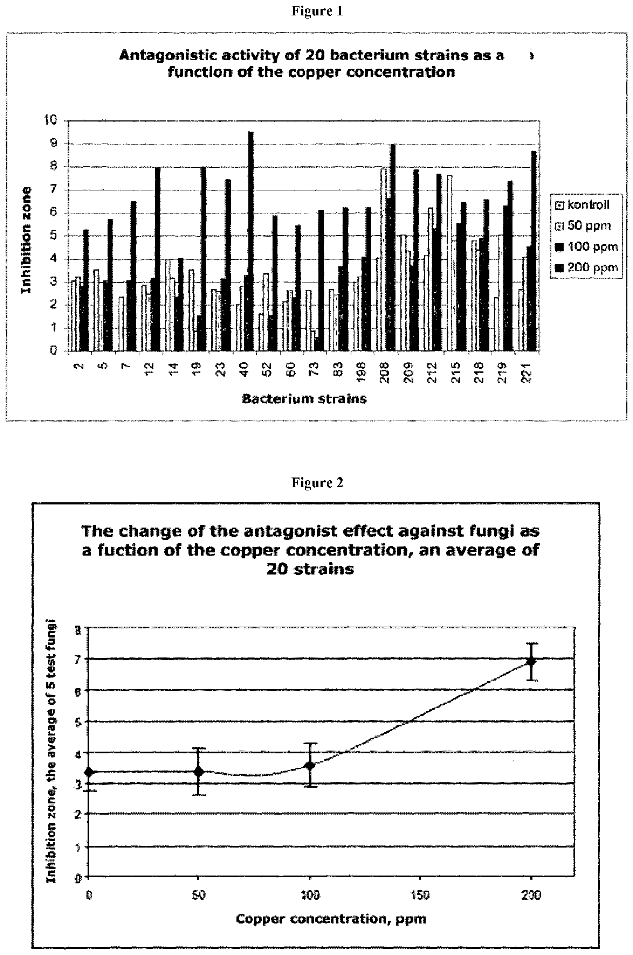

The copper-ion sensitivity of the strains showing excellent antagonism in the widest spectrum was determined by culturing medium dilution method (Fig. 1). Then strains were prepared with a copper-ion tolerance exceeding 200 µg/ml by selection for spontaneous copper-resistance.

The test was made with 20 pre-selected strains: B2, B5, B7, B12, B14, B19, B23, B40, B52, B60, B73, B83, B198, B208, B209, B212, B215, B218, B219, B221. The strains were maintained on peptone culturing medium at +5 °C temperature, for prolonged shelf life on YDC culturing medium, also at +5 °C temperature. The tests, as the majority of the following tests, were made on peptone culturing medium or liquid. The copper tolerance of the strains was tested on a culturing medium containing 10, 20, 40, 100 and 200 ppm copper. The copper was admixed to the culturing medium in the form of copper-sulphate, starting from 10000 ppm stock solution, calculating the concentration for the copper active agent. The fresh culture of the bacteria was used to prepare a 10⁶/ml suspension, and 100 µl of this was transferred into 9 cm Petri dishes. The evaluation was made continuously, 2-3 days afterwards. In the test made in the culturing liquid 10 µl of the bacterium suspension with the same concentration was added to 10 ml culturing liquid. The evaluation was made 2 days afterward, by measuring of the optical density (660 nm), and confirmed by Bürker camera. The incubation was done in each case at a temperature of 22-25 °C, in dark.

All 20 strains grew both on the culturing medium and in the culturing liquid even with 200 ppm copper concentration, and no significant difference was revealed as compared to the growth at lower copper concentration.

In the following tests the copper concentration of the culturing medium was increased to 400 and 800 ppm, respectively. At 400 ppm the B5, B198, B208, B219 and B221 strains showed no growth anymore, the others still grew in a small compass. The 800 ppm concentration resulted in complete inhibition. At the same time, transferring to a culturing medium of 400, then 800 ppm copper concentration, colonies from each strain were isolated which were able to grow in such a high concentration, and they could be maintained in a culturing medium with said copper concentration. As the 400 and in particular 800 ppm copper concentration is higher than the value necessary to inhibit the growth of plant pathogen fungi and bacteria, further, which may appear in soils, it was not reasonable to isolate strains tolerant with even higher copper concentration. The copper tolerant strains were maintained on a culturing medium containing 800 ppm copper at +5 °C temperature.

### The antagonism against plant pathogens, the effect of copper on the antagonism

The tests were made with the above 20 bacterium strains, against the following plant pathogens:
*Pseudomonas syringae* (5 strains)
*Pythium ultimum*
*Phytophthora infestans* (3 strains)
*Sclerotinia sclerotiorum*
*Fusarium oxysporum f.sp. lycopesici*
*Alternaria solani*
*Rhizoctonia solani*

The antagonism tests were made on a peptone agar, on PDA, which is more suitable for fungi, and in case of *Phytophthora* strains on pea agar, supplementing the culturing medium with 0, 50, 100 and 200 ppm copper. The antagonist bacterium strains were inoculated starting from a fresh culture, using a 5 mm diameter filter paper disc, into one third of the 9 cm culturing medium disc, at the same time, in front of them into the second third of the culturing medium disc the plant pathogen *Pseudomonas* strains were inoculated by a similar method, furthermore, the fungi were inoculated using a 5 mm diameter mycelium disc cut off the fresh culture. The incubation took place in each case at a temperature of 22 - 25 °C, in dark.

The evaluation was started 2 days afterwards, and was made continuously, measuring the appeared inhibition zones. The evaluation of the results was done by one and two factor analysis of variance.

From the results in can be seen that there were significant differences between the antagonist bacterium strains regarding the antagonism showed against the fungi pathogens (*Pythium* and genuine fungi), however, in the culturing medium containing copper this difference was insignificant (p=0.05). At the same time the difference grew further upon the addition of 50 ppm copper, and 100 and 200 ppm resulted in significant difference between the strains (Fig. 1).

The addition of copper slightly increased the antagonist activity, however, the difference on average of five test fungi (*P. ultimum, S. sclerotiorum, A. solani, F. oxysporum* and *R. solani*) was significant only at 200 ppm copper concentration (Fig. 2). At the same time, it is worth mentioning that this concentration significantly increased the growth of the test fungi, and made it difficult to evaluate the results. In cases of B2, B5, B7, B12, B19, B23, B40, B52, B60, B73, B83, B 198, B208, B209, B219 and B221 strains a definitely stronger antagonist effect was experienced within such circumstances, however, at lower concentrations (50 and 100 ppm) this phenomenon was seen only with the B208, B219 and B221 strains (Fig. 1).

Considering the average sensitivity of the above-mentioned five test fungi against the antagonists, there were of course significant differences, and these differences were significant at every copper concentration, and even without copper. *F. oxysporum* and *R. solani* were proved to be the least sensitive, in these cases the sensitivity was not even increased by the elevation of the copper concentration. *A. solani* was moderately sensitive as well, but the copper concentration of 200 ppm resulted in a significant increase in the sensitivity. *P. ultimum* and the tested *S. sclerotinia* strains were more sensitive, especially with high copper concentration.

The reaction of the tested plant pathogen *P. syringae* bacteria was significantly different from the pathogen fungi. There was not any significant difference in the effectiveness of the 20 antagonist bacteria considering the average of the 5 *P. syringae* strains. Similarly, there was no significant difference between the sensitivity of the different *P. syringae* strains. Unlike the experiences with the plant pathogen fungi, the copper content of the culturing medium definitely decreased the effectiveness of the antagonists. The difference experienced was significant only at 100 and 200 ppm copper concentration. (The copper sensitivity of the plant pathogen *P. syringae* strains was approximately the same as that of the 20 antagonist bacteria: all increased at 200 ppm concentration, and none of them at 400 ppm.)

The *Phytophthora infestans* strains involved in the test were more sensitive to the copper ions as compared with the other pathogens, they did not show growth anymore at 100 ppm concentration, therefore the tests were made only at 50 ppm concentration. There was significant difference between the efficacies of the antagonist bacteria, however, the presence of copper did not result in significant change (it was similar to the other tested fungi, where there was no clear change in the character of the antagonist activity at 50 ppm copper concentration).

As to the influence of the copper ions on the efficacy of the antagonists, it was established that the enhancement of the efficacy that may be used in the further tests was experienced only in cases of plant pathogen fungi and only at a relatively high (200 ppm) copper ion concentration.

### Greenhouse and field tests with the promising bacterium strains

Greenhouse and field treatment tests were made with the copper-resistant strains proven to be the best antagonist. The tests aimed at clarifying the rhysosphere tolerance of the antagonist strains in tomato and pepper culture (culturing methods using soil or using no soil), and in other vegetable cultures (cabbage, lettuce), and especially the very important question of how the plant tolerates the treatment with the bacterium. Using quantitative culturing from the rhizomes of the treated plants it was clarified if the bacterium strain is incorporated in the plant, if it makes colonies endogenously without adversely affecting the development of the plant. The tests were run with 20 strains possessing excellent *in vitro* antagonism spectrum.

The tests were made in two periods, using 2x10 bacterium strains. The strains to be tested (members of the *Bacillus, Pseudomonas, Pantoea* genus) were diluted to the required concentration, and the production boxes and the rock wool sowing platform were irrigated with this solution immediately after sowing. The further tests were made with young plants already having leaves. The plant samples were collected at two dates, where the leaves of the selected plants were counted, and the plants were collected without their roots and their fresh green mass was weighed. The tests were made with pepper and tomato species, in soil and rock wood production system, considering the irrigation with three different bacterium concentrations. In cases of cabbage and lettuce the tests were set only to the soil production method. All tests were made in four sets, in random block arrangements.

10 *Bacillus* strains have proven to be the best.

### Greenhouse and field tests II. Testing of the ability to induce resistance

Regarding the antagonist bacterium component according to our planned compositions it is an important requirement that it should provide protection against the bacterial and fungal pathogens not only at the rhizosphere, but also at the organs of the plants above the soil through the activation of the inducible resistance mechanisms of the plant. The tests were made with tomato and pepper. The roots of the young plants were treated with the suspension of the antagonist bacterium strains, and after planting, when different intervals in time have passed, artificial infections were provoked on the leaves with a cell and conidium suspension of the plant pathogen bacteria and fungi. Furthermore, it was evaluated how the level of the chemicals playing important role in the induced resistance changes in the treated plants as compared with the control (reference) plants.

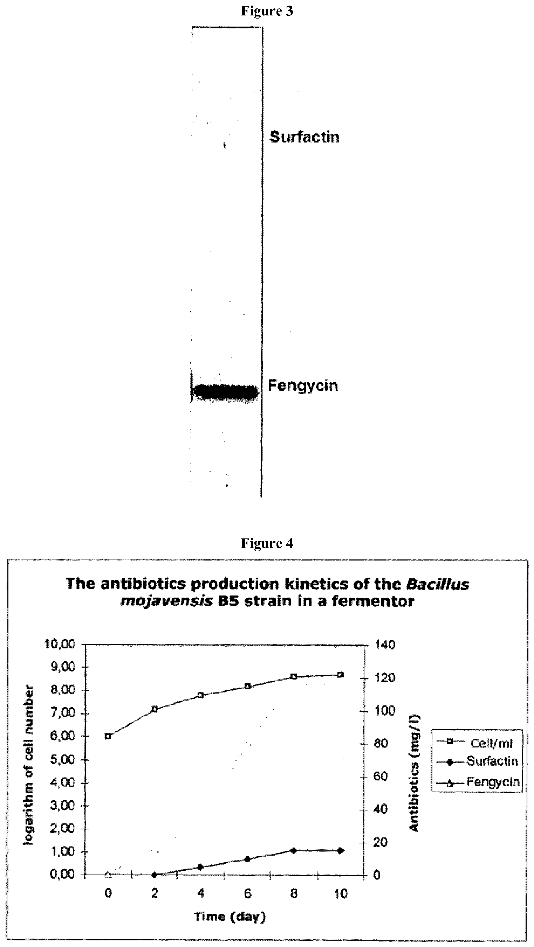

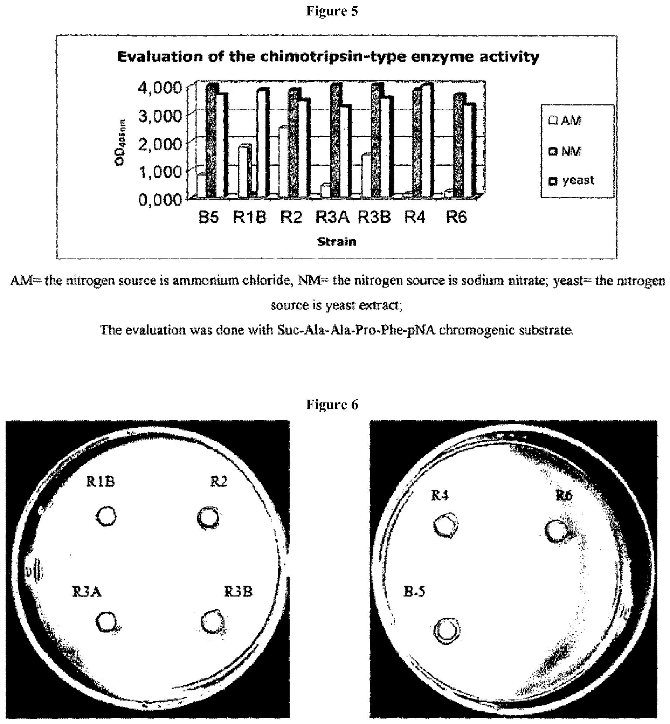

Our excellent antagonist B5 *Bacillus* strain had the ability to induce the self-protection mechanism of the plant [Systemic Acquired, Resistance, (SAR)]. The R3B copper resistant mutant spontaneously appearing after transferring of the *Bacillus mojavensis* B5 strain onto a culturing medium containing 400 µg/ml copper-sulphate was proven to be the best. This strain possesses wide spectrum and excellent antagonist abilities probably for the reason of secreting in large amount an antifungal depsi-peptide antibiotics, fengycin (Fig. 4), and the constitutive chimotripsin type protease, which enhances the effect through synergism (Fig. 5).

### Evaluation of the antibiotics and chimotripsin production of the Bacillus mojavensis B5 strain and the copper resistant mutants made therefrom

The strains were maintained by weekly cross inoculation, on YEG culturing plates (0.2% glucose, 0.2% yeast extract, 2% baktoagar). For the antibiotics production tests mainly minimal culturing solution was used suitably changing the used carbon and nitrogen source, furthermore the concentration of the two trace elements (iron and copper). This culturing solution, unlike e.g. a yeast extract based culturing solution, does not contain amino acids and peptides, thus the analysis and purification of the antibiotics mixtures secreted into the fermenting liquid can be made more easily.

Based on the preliminary production experiences, the following GGM culturing solution was used:

| | |
|---|---|
| Glucose | 1% |
| Glutaminic acid Na salt | 0.5% |
| KH₂PO₄ | 0.1 % |
| K₂HPO₄ | 0.1% |
| MgSO₄ x 7H₂O | 0.05% |
| KCl | 0.1 % |
| FeSO₄ x 7H₂O | 10 mg/l |
| CuSO₄ x 5H₂O | 1 mg/l |

20 ml culturing solution was measured into 50 ml Erlenmeyer flasks. After inoculation the culturing took place on an orbital shaking device for 6 days, at 180 rpm and 25 °C temperature.

The cell density of the cultures was determined by measuring the absorption at 620 nm. In case of the *Bacillus* strains a 0,1 OD value is equivalent with a 10⁷ cell/ml concentration. Thereafter the bacterium cells were settled by centrifuging at 8000 G for 10 minutes, and the supernatant was transferred to a beaker, then the pH of the fermented liquids was set to 2 using 10% hydrochloric acid (0,4 ml to 20 ml fermented liquid). The precipitated fermented liquids were incubated at 5 °C temperature overnight, to complete the precipitation. Then the precipitate was settled by centrifugation, and dissolved in 1 ml 96 % ethanol.

### The quantitative determination of the antibiotics from the ethanol preparations

The fengycin molecule contains 2 tyrosine molecules, which however shows a strong absorbance at 280 nm. Thus, the antibiotics content other than surfactin of the antibiotics preparations may be estimated by measuring at 280 nm. Thus, all OD of the ethanol preparations diluted to 10 times of their original concentration was measured at 280 nm.

### Thin layer chromatography (TLC) of the secreted antibiotics

In our preliminary studies, as well as in the tests of the novel strains in the thin layer chromatographic analysis of the strains a glutaminic acid/glucose culturing solution was used, as in cases of most strains this provided the sufficient yield of antibiotics. Fig. 3 shows the results of the TLC analysis of the fermented liquid of the B5 strain.

In the course of the pest control application it is extremely important to use the suitable culturing medium when preparing the cultures. The results prove that the above-mentioned glutaminic acid/glucose culturing solution should be used, as the culture may be diluted even to 10-20 times of its original volume, and even in this case the fengycin concentration/ml is 10-12 mg/l, which achieves a complete inhibition of the majority of the plant pathogen fungi, and is sufficient to activate the induced resistance mechanism in the treated plant.

### The antagonist ability of the B. mojavensis B5 strain and the copper resistant mutants spontaneously produced therefrom

The strains were cultured on a yeast extract culturing medium for 48 hours. The inhibition of *Pseudomonas syringae, Clavibacter michiganensi* and *Xanthomonas campestris* by the B5 strain and its copper resistant mutants was tested. It can be seen in the table that the best inhibiting effect is possessed by the R3B copper resistant mutant.

| **Inhibition zone (mm)** | | | | | | | |
|---|---|---|---|---|---|---|---|
| | B-5 | R1B | R2 | R3A | **R3B** | R4 | R6 |
| ***Pseudomonas syringae*** | 3.0 | 2.0 | 4.0 | 3.0 | **5.0** | 2.0 | 3.0 |
| ***Clavibacter michiganensis*** | 6.0 | 2.0 | 3.0 | 6.0 | **7.0** | 5.0 | 4.0 |
| ***Xanthomonas campestris*** | 7.0 | 5.0 | 6.0 | 7.0 | **7.0** | 7.0 | 8.0 |

The copper-resistant B5 strains' inhibition effects on *Fusarium osysporum* was compared to the parent strain. The table shows that in this case also the R3B copper resistant mutant possesses the best efficacy. Therefore, we seek protection for this strain.

| **Inhibition zone (mm)** | | | | | | | |
|---|---|---|---|---|---|---|---|
| | B-5 | R1B | R2 | R3A | **R3B** | R4 | R6 |
| ***Fusarium oxysporum*** | 3.5 | 3.5 | 3.0 | 4.0 | **4.0** | 3.5 | 4.5 |

### Industrial applicability of the invention

It is a benefit of the present invention that the isolated *Bacillus mojavensis* R3B mutant strain induces a strong antagonist effect against pathogens, and also carries copper-resistance and has the ability to activate an induced resistance in plants against the pathogens. Due to its copper-resistant feature, it may be used together with copper containing pesticides, thus may effect an integrated pest control.

## Claims

1. Use of *Bacillus mojavensis* R3B copper resistant mutant strain deposited under # NCAIM (P) B 001389 according to the Budapest Treaty for exerting antagonism against pathogens selected from the group of *Xanthomonas vesicatoria, Pseudomonas syringae* and *Clavibacter michiganensis* plant pathogen bacteria and *Pythium debaryanum* and *Alternaria alternata* plant pathogen fungi.

2. The use according to Claim 1, wherein the pathogens are pathogens of green vegetables.

3. The use according to Claim 2, wherein the green vegetables are selected from the group of tomato, pepper, lettuce and cabbage.

4. Use of a composition as a pesticide, which composition contains a culture of the *Bacillus mojavensis* R3B copper resistant mutant strain deposited under # NCAIM (P) B 001389 according to the Budapest Treaty, and optionally a copper-containing pesticide and optionally an excipient.

5. The use according to Claim 4, wherein the copper-containing pesticide is selected from the group of copper-sulphate, copper-oxyquinolate, copper-oxide, copper-hydroxide and copper-oxy-chloride.

6. The use according to Claim 4 or 5, wherein the composition is in an aqueous suspension, suspension concentrate, capsulated concentrate, emulsion forming liquid spray, granule, granule dispersible in water, microgranule or water soluble powder dose formulation.

7. A process for controlling pathogens selected from the group of *Xanthomonas vesicatoria, Pseudomonas syringae* and *Clavibacter michiganensis* plant pathogen bacteria and *Pythium debaryanum and Alternaria alternata* plant pathogen fungi, **characterized in that** the *Bacillus mojavensis* R3B copper resistant mutant strain deposited under # NCAIM (P) B 001389 according to the Budapest Treaty or the composition thereof is applied to a plant.

8. The process according to Claim 7, wherein the plant is a vegetable.

9. The process according to Claim 8, wherein the vegetable is selected from the group of tomato, pepper, lettuce and cabbage.

10. The process according to any of Claims 7 to 9, **characterized in that** the *Bacillus mojavensis* R3B copper resistant mutant strain deposited under # NCAIM (P) B 001389 according to the Budapest Treaty or the composition thereof is applied to the
a) seeds of the plant,
b) roots of the plant,
c) stems of the plant,
d) leaves, flowers, foliage or fruits of the plant,
is mixed to
e) the irrigation water of the plant and/or
is sprayed
f) to the plant.

## Patentansprüche

1. Verwendung des kupferresistenten mutanten Stammes *Bacillus mojavensis* R3B, welcher gemäß dem Budapester Abkommen unter der Nr. # NCAIM (P) B 001389 hinterlegt ist, zur Ausübung einer antagonistischen Wirkung gegen pathogene Erreger, ausgewählt aus der Gruppe *Xanthomonas vesicatoria, Pseudomonas syringae* und *Clavibacter michiganensis* pflanzenpathogene Bakterien und *Pythium debaryanum* und *Alternaria alternata* pflanzenpathogene Pilze.

2. Die Verwendung gemäß Anspruch 1, wobei die pathogenen Erreger Erreger von grünem Gemüse sind.

3. Die Verwendung gemäß Anspruch 2, wobei das grüne Gemüse ausgewählt ist aus der Gruppe Tomaten, Paprika, Salat und Kohl.

4. Verwendung einer Zusammensetzung als Pestizid, welche Zusammensetzung eine Kultur des kupferresistenten mutanten Stammes *Bacillus mojavensis* R3B, welcher gemäß dem Budapester Abkommen unter der Nr. # NCAIM (P) B 001389 hinterlegt ist, und gegebenenfalls ein kupferhaltiges Pestizid und gegebenenfalls einen Hilfsstoff enthält.

5. Die Verwendung gemäß Anspruch 4, wobei das kupferhaltige Pestizid aus der Gruppe Kupfersulfat, Kupferoxichinolat, Kupferoxid, Kupferhydroxid und Kupferoxichlorid ausgewählt ist.

6. Die Verwendung gemäß Anspruch 4 oder 5, wobei die Dosierungsform der Zusammensetzung wässrige Suspension, Suspensionskonzentrat, Konzentratkapsel, emulgierbares flüssiges Sprühmittel, Granulat, in Wasser dispergierbares Granulat, Mikrogranulat oder wasserlösliches Pulver ist.

7. Ein Verfahren zur Kontrolle von pathogenen Erregern ausgewählt aus der Gruppe *Xanthomonas vesicatoria, Pseudomonas syringae* und *Clavibacter michiganensis* pflanzenpathogene Bakterien und *Pythium debaryanum* und *Alternaria alternata* pflanzenpathogene Pilze, **dadurch gekennzeichnet, dass** der kupferresistente mutante Stamm *Bacillus mojavensis* R3B, welcher gemäß dem Budapester Abkommen unter der Nr. # NCAIM (P) B 001389 hinterlegt ist, oder eine Zusammensetzung dessen auf eine Pflanze aufgebracht wird.

8. Das Verfahren gemäß Anspruch 7, wobei die Pflanze ein Gemüse ist.

9. Das Verfahren nach Anspruch 8, wobei das Gemüse aus der Gruppe Tomaten, Paprika, Salat und Kohl ausgewählt ist.

10. Das Verfahren nach einem der Ansprüche 7-9, **dadurch gekennzeichnet, dass** der kupferresistente mutante Stamm *Bacillus mojavensis* R3B, welcher gemäß dem Budapester Abkommen unter der Nr. # NCAIM (P) B 001389 hinterlegt ist, oder eine Zusammensetzung dessen auf
a) die Samen der Pflanze,
b) die Wurzeln der Pflanze,
c) die Stiele der Pflanze,
d) die Blätter, die Blüten, das Laub der Pflanze oder die Früchte der Pflanze aufgebracht wird,
e) dem Bewässerungswasser der Pflanze
zugegeben wird und/oder
f) auf die Pflanze
gesprüht wird.

## Revendications

1. Utilisation de la souche mutante résistante au cuivre de *Bacillus mojavensis* R3B déposée sous # NCAIM (P) B 001389 selon le Traité de Budapest pour exercer un antagonisme contre des pathogènes choisis dans le groupe des bactéries phytopathogènes *Xanthomonas vesicatoria, Pseudomonas syringae* et *Clavibacter michiganensis* et des champignons phytopathogènes *Pythium debaryanum* et *Alternaria alternata.*

2. Utilisation selon la revendication 1, dans laquelle les pathogènes sont des pathogènes des légumes.

3. Utilisation selon la revendication 2, dans laquelle les légumes sont choisis dans le groupe de la tomate, du poivron, de la laitue et du chou.

4. Utilisation d'une composition comme pesticide, laquelle composition contient une culture de la souche mutante résistante au cuivre de *Bacillus mojavensis* R3B déposée sous le # NCAIM (P) B 001389 selon le Traité de Budapest, et éventuellement un pesticide contenant du cuivre et éventuellement un excipient.

5. Utilisation selon la revendication 4, dans laquelle le pesticide contenant du cuivre est choisi dans le groupe du sulfate de cuivre, de l'oxyquinoléate de cuivre, de l'oxyde de cuivre, de l'hydroxyde de cuivre et de l'oxychlorure de cuivre.

6. Utilisation selon les revendications 4 ou 5, dans laquelle la composition est dans une forme de dosage d'une suspension aqueuse, d'un concentré de suspension, d'un concentré encapsulé, d'un agent de pulvérisation liquide émulsionnable, d'un granulé, d'un granulé dispersible dans l'eau, d'un microgranulé ou d'une poudre soluble dans l'eau.

7. Procédé pour contrôler des pathogènes choisis dans le groupe des bactéries phytopathogènes *Xanthomonas vesicatoria, Pseudomonas syringae* et *Clavibacter michiganensis* et des champignons phytopathogènes *Pythium debaryanum* et *Alternaria alternata,* **caractérisé en ce que** la souche mutante résistante au cuivre de *Bacillus mojavensis* R3B déposée sous le # NCAIM (P) B 001389 selon le Traité de Budapest, ou une composition de celle-ci est appliquée à une plante.

8. Procédé selon la revendication 7, dans lequel la plante est un légume.

9. Procédé selon la revendication 8, dans lequel le légume est choisi dans le groupe de la tomate, du poivron, de la laitue et du chou.

10. Procédé selon l'une quelconque des revendications 7 à 9, **caractérisé en ce que** la souche mutante résistante au cuivre de *Bacillus mojavensis* R3B déposée sous le # NCAIM (P) B 001389 selon le Traité de Budapest ou la composition de celle-ci est appliquée aux
a) graines de la plante,
b) racines de la plante,
c) tiges de la plante,
d) feuilles, aux fleurs, au feuillage ou aux fruits de la plante,
est mélangée à
e) l'eau d'irrigation de la plante et/ou
est pulvérisée
f) sur la plante.
